# EUROPEAN PATENT APPLICATION

(11) **EP 4 098 219 A1**
(43) Date of publication of application: **07.12.2022**
(21) Application number: 21197353.2
(22) Date of filing: 17.09.2021
(51) Int. Cl.: A61B 34/10, A61F 2/86, G06N 3/02, A61B 90/00

(54) **MACHINE LEARNING BASED SYSTEMS AND METHODS FOR CREATING PERSONALIZED ENDOVASCULAR STENTS AND STENT GRAFTS**

(30) Priority: 04.06.2021 US 202163196954 P
(71) Applicant: Inteneural Networks Inc., Chicago, IL 60610 (US)
(72) Inventor: Kris, Siemionow, Chicago (US); Kraft, Marek, Poznan (PL); Mikolajczak, Michal, Poznan (PL); Pieczynski, Dominik, Tulce (PL); Pawlak, Mikolaj, Poznan (PL); Klimont, Michal, Poznan (PL); Lewicki, Paul, Tulsa (US)
(74) Representative: Kancelaria Eupatent.pl Sp. z.o.o

(57) **Abstract**

A method for creating a personalized stent or stent graft (10) for a blood vessel (20) with a saccular aneurysm (21), the method comprising: receiving (206) a 3D model of the blood vessel (20) with the saccular aneurysm (21); and generating (207) a model of a personalized stent or stent graft (10) that comprises a net (11) shaped to fit along internal walls of the blood vessel (20) and a covering (12) positioned with respect to the net (11) such as to cover an ostium of the aneurysm (21).

## Description

### TECHNICAL FIELD

The present disclosure generally relates to machine learning systems and methods suitable for creating personalized endovascular stents and stent grafts.

### BACKGROUND

Placing an endovascular stent or stent graft is particularly challenging in case of cranial aneurysms, wherein the stent graft should not occlude collateral circulation (also called a perforator).

Saccular intracranial aneurysms are defined as dilatations of the cerebral artery with two crucial parts ― the aneurysm's neck and the aneurysm's sack. An aneurysm rupture has a high mortality; therefore, rupture prevention is the main goal in treating intracranial aneurysms. Excluding the aneurysm from blood inflow leads to clot formation inside the aneurysm sack and prevents future rupture. There are multiple methods for achieving this. One of the possible treatment methods is coil embolization, i.e. inserting foreign bodies that promote clot formation inside the aneurysm. Another approach involves placing a stent (i.e. metal scaffold) over the aneurysm neck to limit the inflow. Yet another approach is use of flow diverters, which is similar to using stents, but incorporates a much denser structure that decreases the inflow considerably. Blood flow limiting devices (i.e. stents and flow diverters) are placed over an aneurysm's neck and adjacent artery segment, influencing the blood flow in small arteries (perforators).

Ideally, the blood flow limiting device should only cover the aneurysm neck and spare all the neighboring perforating vessels and collateral blood supply. This device is currently not available due to limitations in the stent design and architecture, as well as stent positioning capabilities of the physician.

Recent advances in imaging technology and machine learning algorithms allow for precise segmentation of the vascular anatomy. This segmentation can then be used to create a 3D model of the aneurysm and surrounding collateral circulation. This information can then be used to design a patient specific endovascular stent that takes the patient's unique anatomy into account ensuring that the above mentioned limitations are avoided. Such a personalized stent may be best suited to be deployed with an image guidance system that once again takes into account the individual patient's unique anatomy into consideration. Since there many variables that need to be taken into account when designing and deploying a personalized stent, machine learning algorithms are well suited for anatomy segmentation, identification of aneurysm characteristics, assessment of collateral circulation, creation of a 3D model of the aneurysm and preparation of stent design options to the treating physician.

### SUMMARY OF THE INVENTION

Therefore, there is a need for creating personalized endovascular stents and stent grafts that would solve at least some of the problems discussed above.

The invention described herein is particularly useful for creating personalized endovascular stents and stent grafts into arteries with aneurysm and collateral circulation, such that the stent or stent graft does not occlude the collateral circulation.

The invention describes a device that can exclude the intracranial aneurysm from blood circulation by covering the aneurysm ostium, which prevents its rupture. This is achieved by a precisely designed stent with a not permeable area that matches the aneurysm's neck in dimensions and location and that can be placed such as to cover the aneurysm ostium. Limiting the area of dense stent covering prevents neighboring perforators occlusion.

For this stent design to achieve the above described goals the dimensions as well as the 3D morphology of the aneurysm ostium must be precisely described. This information could previously be done by hand by a human operator. This process however is fraught with many potential errors as it is challenging for a human operator to perform this task on 2D image slices. Using CNN to perform vessel segmentation and aneurysm detection is the first step which allows for detailed analytics of the region of interest. Once the region of interest is identified, the information about the aneurysm ostium can be transferred to the stent design process. The stent is to be designed in such a way as to have its impermeable part cover only the ostium with minimal to no coverage of the healthy vessel.

The invention relates to a method for creating a personalized stent or stent graft for a blood vessel with a saccular aneurysm, the method comprising: receiving a 3D model of the blood vessel with the saccular aneurysm; and generating a model of a personalized stent or stent graft that comprises a net shaped to fit along internal walls of the blood vessel and a covering positioned with respect to the net such as to cover an ostium of the aneurysm.

The method may further comprise generating the 3D model of the blood vessel with the saccular aneurysm by: reading an input 3D image representing an anatomic volume including the blood vessel with the saccular aneurysm; pre-processing the input 3D image to remove at least some of elements other than the blood vessel with the saccular aneurysm; and creating the 3D model of the blood vessel with the saccular aneurysm by means of a dedicated algorithm.

The dedicated algorithm for creating the 3D model of the blood vessel can be at least one artificial neural network.

Creating the 3D model of the blood vessel may comprise a pipeline of: segmenting the pre-processed input 3D image to create a scaffold around a shape of the vessels to output a 3D model that represents a binary segmentation of a vessels tree; detecting a bounding box for the aneurysm; and segmenting the aneurysm and a neighboring vessel segment to output the 3D model of the blood vessel with the saccular aneurysm.

The input 3D image can be represented by Time of Flight Magnetic Resonance Angiographs (TOF-MRAs), CT (Computed Tomography) angiography or flat detector CT angiography images.

The method may further comprise creating the personalized stent or stent graft based on the 3D model of the blood vessel with the saccular aneurysm.

The invention also relates to a personalized stent or stent graft for a blood vessel with a saccular aneurysm, the stent or stent graft comprising a net shaped to fit along internal walls of the blood vessel and a covering positioned with respect to the net such as to cover an ostium of the aneurysm.

These and other features, aspects and advantages of the invention will become better understood with reference to the following drawings, descriptions and claims.

### BRIEF DESCRIPTION OF DRAWINGS

Various embodiments are herein described, by way of example only, with reference to the accompanying drawings, wherein:
Fig. 1 shows a stent graft according to an embodiment of the invention;
Fig. 2 shows a procedure for creating a personalized stent or stent graft;
Fig. 3 shows the structure of a computer system for implementing the method of Fig. 2;
Fig. 4 shows examples of input data and output model.

### DETAILED DESCRIPTION

The following detailed description is of the best currently contemplated modes of carrying out the invention. The description is not to be taken in a limiting sense, but is made merely for the purpose of illustrating the general principles of the invention.

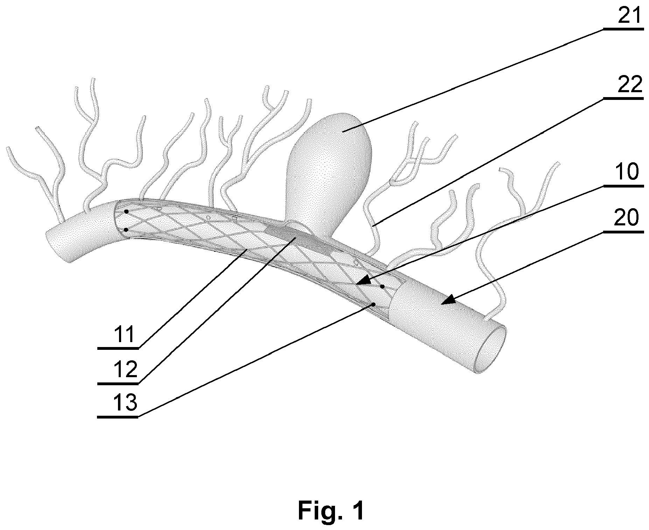

The following embodiment will be presented with respect to brain arteries, but it is applicable to any blood vessels wherein saccular aneurysm can occur, such as splenic artery aneurysms, renal artery arteries, popliteal artery arteries. Pseudoaneurysms are also eligible for treatment by this method. The aim of the method and system presented herein is to provide a personalized stent 10 or a personalized stent graft to be placed within a blood vessel 20 with aneurysm 21, wherein the stent or stent graft 10 includes a net 11 and a covering 12 positioned such as to cover the ostium of the aneurysm 21, but not to occlude collateral circulations (perforators) 22, as shown schematically in Fig. 1. The stent is defined as a scaffold placed on the inside of the vessel, whereas the stent graft is a device which in addition to a similar structure, has an impermeable coating fixed on the scaffold, placed between the vessel's lumen and the vessel's wall.

The stent or stent graft 10 further comprises a plurality of markers 13 to facilitate positioning of the stent 10 in appropriate location within the blood vessel 20.

The stent or stent graft 10 may be of any known type, such as self-expandable, nitinol, braided stents.

The markers 13 can be electromagnetic markers that are detectable by fluoroscopy. They can be positioned at a plurality of locations, for example they can be attached to the net 11 at the ends of the stent 10 (to facilitate positioning of the whole stent 10) and/or attached to the net 11 at the boundary of the covering 12 (to facilitate positioning of the covering 12 at ostium of the aneurysm).

The covering 12 can be a synthetic covering, for example from a not permeable fabric or a dense metal scaffold, which is positioned at the part of the stent which covers aneurysm's ostium. The covering 12 shall be positioned at least at the location of the aneurysm 21 such as to cover the aneurysm 21, this covering shall be positioned only at the ostium preventing blood flow into the aneurysm.

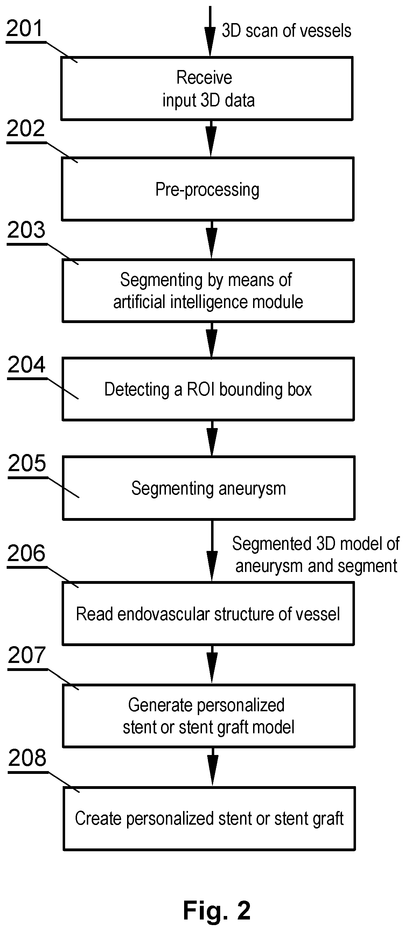

Fig. 2 shows a procedure for creating, inserting and positioning the stent or stent graft 10.

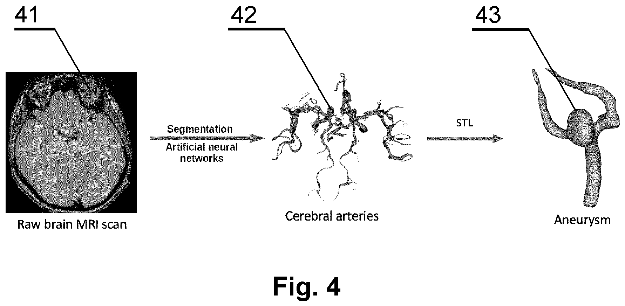

First, in step 201, an input 3D image is read that represents anatomic volume including the blood vessel 20 with a saccular aneurysm 21, into which a stent needs to be positioned. For example, the input image may be represented by Time of Flight Magnetic Resonance Angiographs (TOF-MRAs), CT (Computed Tomography) angiography or flat detector CT angiography. For example, the input image can be provided in the NIFTI format or DICOM format. An example of an input image 41 is presented on Fig. 4.

In step 202, pre-processing of the input 3D image is performed to remove at least some of the unnecessary elements (such as parts of the skull if the image relates to brain arteries) other than the blood vessel 20 with a saccular aneurysm 21 and to enhance image readability, such as bias field correction, clipping signal outliers values or skull stripping (using pretrained for this task CNN).

In step 203, the pre-processed input 3D image is segmented to create a scaffold around the shape of the vessels, to provide an output 3D model 42 (as shown in Fig. 4) that represents the binary segmentation of vessels tree.

Next, in step 204, a region of interest (ROI), i.e. a bounding box for aneurysm is detected.

Finally, in step 205 detailed segmentation of aneurysm and neighboring vessel segment (in which the stent is to be placed) is performed.

Each of steps 203-205 can be performed by a dedicated algorithm, in particular a neural network, such as a convolutional neural network. They artificial neural networks shall be designed for 3D models, as spatial context is crucial for this particular task.

For example, in step 203, a neural network may be trained with training data including binary vessels tree labels desired as model output, while the input shall include a 3D image from a medical scan, preprocessed appropriately as described in step 202. The input data might be also enhanced with responses of special filters dedicated for detection of vessels and tube-like structures such as Frangi or Jerman filter. Those are obtained using preprocessed input data and provided as separate concatenated channels.

For example, in step 204, an object detection network can be used to detect aneurysms. Training data for this network includes 3D preprocessed image, as well as obtained binary vessels segmentation as input, while the output is a set of coordinates of the bounding box of detected aneurysm. The bounding boxes are later used to select a ROI, which is fed to another segmentation network, responsible for segmentation of the aneurysms.

The pipeline of steps 203-205 allows to obtain more refined/precise aneurysms segmentations from local cubes of image, which are later placed/marked at the original coordinate system.

In step 206, the endovascular structure of the blood vessel to be stented is read, including data on its nearby aneurysm and collateral circulation, such as to determine the endoluminal shape of the blood vessel and the aneurysm.

In step 207, a personalized stent or stent graft model is automatically generated, such as shown in Fig. 1, for the blood vessel 20 to be stented, with a net 11 shaped to fit along the internal walls of the blood vessel 20 and a covering 12 positioned with respect to the net such as to cover the ostium of the aneurysm 21, but does not occlude the collateral circulation 22. First, the ROI to operate on is restricted. This is done by creating a morphological skeleton of an acquired vessels tree, detecting branching points in it, and splitting the tree into segments located between them. A vessel segment on which the ostium of the aneurysm is located is selected for future processing. If the aneurysm is located close to the branching point, all segments directly connected to the branching are used instead of individual segment in order to provide a better context (as referenced by 43 in Fig. 4). Then a set of features normally affecting the human expert/clinician decision about stent to be used is derived. Distance transform, followed by masking its results with acquired later skeleton for selected segment is used to obtain information about segment radius. The volume and shape globularity metrics of the aneurysm are computed by using voxels labeled as such and corresponding voxel spacing information from original scan metadata. From labels of both vessel tree and aneurysm, the set of voxels being the boundary between them is acquired using morphological operations (edges of both segment and aneurysms objects are detected and dilated, then their intersection is obtained)). Total area of this boundary as well as the shape and measurements of the aneurysm neck are computed using voxels spacing information. Those features combined are input to the next model which outputs information on how long the scaffold of the stent and how dense the not permeable area of it should be. In one embodiment, the model itself can be a rule-based expert system, with rules based on knowledge provided by multiple clinicians. In another embodiment, the model itself can be a machine learning regression model, trained with analogical input/output data as described. Once the described parameters are available, the stent model itself is generated based on them and the previously selected vessel segment geometry. The resulting model can be exported to common 3D formats such as.stl,.ply or. obj.

In step 208, the personalized stent or stent graft is created based on the created model, by means of a 3D printing process or any other suitable stent creating process. The stent or stent graft 1 includes the covering and markers according to the model.

That stent or stent graft can be later on inserted and positioned within the blood vessel according to known methods, with the aid of the vessel model output in step 203, such that it is appropriately located within the blood vessel. Insertion and optimal placement of the personalized graft can be performed by matching the position of the graft with reference markers to a pre-surgical model of the vasculature created by the machine learning algorithm in step 203. An electromagnetic navigation system can be used to provide appropriate reference parameters to facilitate graft placement.

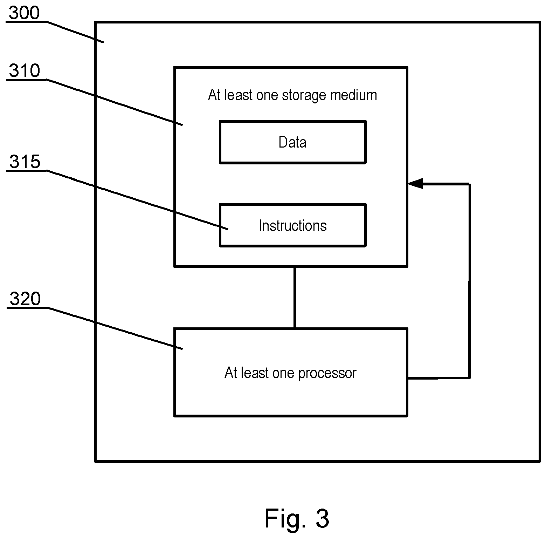

The functionality described herein can be implemented in a computer-implemented system 300, such as shown in Fig. 3. The system may include at least one non-transitory processor-readable storage medium that stores at least one of processor-executable instructions or data and at least one processor communicably coupled to at least one non-transitory processor-readable storage medium. At least one processor is configured to perform the steps of the methods presented herein.

The computer-implemented system 300, for example a machine-learning system, may include at least one non-transitory processor-readable storage medium 310 that stores at least one of processor-executable instructions 315 or data; and at least one processor 320 communicably coupled to the at least one non-transitory processor-readable storage medium 310. At least one processor 320 may be configured to (by executing the instructions 315) to perform the steps of the method of Fig. 2.

While the invention has been described with respect to a limited number of embodiments, it will be appreciated that many variations, modifications and other applications of the invention may be made. Therefore, the claimed invention as recited in the claims that follow is not limited to the embodiments described herein.

## Claims

1. A method for creating a personalized stent or stent graft (10) for a blood vessel (20) with a saccular aneurysm (21), the method comprising:
- receiving (206) a 3D model of the blood vessel (20) with the saccular aneurysm (21); and
- generating (207) a model of a personalized stent or stent graft (10) that comprises a net (11) shaped to fit along internal walls of the blood vessel (20) and a covering (12) positioned with respect to the net (11) such as to cover an ostium of the aneurysm (21).

2. The method according to claim 1, further comprising generating the 3D model of the blood vessel (20) with the saccular aneurysm (21) by:
- reading (201) an input 3D image representing an anatomic volume including the blood vessel (20) with the saccular aneurysm (21);
- pre-processing (202) the input 3D image to remove at least some of elements other than the blood vessel (20) with the saccular aneurysm (21); and
- creating (203-205) the 3D model of the blood vessel (20) with the saccular aneurysm (21) by means of a dedicated algorithm.

3. The method according to claim 2, wherein the dedicated algorithm for creating (203-205) the 3D model of the blood vessel (20) is at least one artificial neural network.

4. The method according to claim 2 or 3, wherein creating (203-205) the 3D model of the blood vessel (20) comprises a pipeline of:
- segmenting (203) the pre-processed input 3D image to create a scaffold around a shape of the vessels to output a 3D model that represents a binary segmentation of a vessels tree;
- detecting (204) a bounding box (ROI) for the aneurysm; and
- segmenting (205) the aneurysm and a neighboring vessel segment to output the 3D model of the blood vessel (20) with the saccular aneurysm (21).

5. The method according to any of claims 2-4, wherein the input 3D image is represented by Time of Flight Magnetic Resonance Angiographs (TOF-MRAs), CT (Computed Tomography) angiography or flat detector CT angiography images.

6. The method according to any of previous claims, further comprising creating (208) the personalized stent or stent graft (10) based on the 3D model of the blood vessel (20) with the saccular aneurysm (21).

7. A personalized stent or stent graft for a blood vessel (20) with a saccular aneurysm (21), the stent or stent graft (10) comprising a net (11) shaped to fit along internal walls of the blood vessel (20) and a covering (12) positioned with respect to the net (11) such as to cover an ostium of the aneurysm (21).
